# FASCICULE DE BREVET EUROPEEN

(11) **EP 2 365 964 B1**
(45) Date de publication et mention de la délivrance du brevet: **18.01.2017**
(21) Numéro de dépôt: 09764489.2
(22) Date de dépôt: 26.11.2009
(51) Int. Cl.: C07C 253/20

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKTIONEN
METHOD FOR MANUFACTURING COMPOUNDS INCLUDING NITRILE FUNCTIONS

(30) Priorité: 04.12.2008 FR 0858264
(43) Date de publication de la demande: 21.09.2011
(73) Titulaire: Rhodia Opérations, 93306 Aubervilliers (FR)
(72) Inventeur: JACQUOT, Roland, 69340 Francheville (FR); MARION, Philippe, 69390 Vernaison (FR)
(74) Mandataire: Chatelan, Florence Anne
(86) Numéro de dépôt international: PCT/EP2009/065872
(87) Numéro de publication internationale: WO 2010/063632

(56) Documents cités:
- EP-A- 0 196 554
- DE-B- 1 268 610
- GB-A- 797 945
- GB-A- 1 397 729
- US-A1- 2005 059 836
- DATABASE CA [Online] CHEMICAL ABSTRACTS SERVICE, COLUMBUS, OHIO, US; 2008, COPE, ARTHUR C. ET AL: "Azelanitrile" XP002533942 extrait de STN Database accession no. 2008:1384858 & ORGANIC SYNTHESES , 34, NO PP. GIVEN CODEN: OSRYAV URL: HTTP://WWW3.INTERSCIENCE.WILEY.COM/CGI-BIN /MRWHOME/104554793/HOME, 1954,

## Description

La présente invention concerne la fabrication de composés comprenant des fonctions nitriles, plus préférentiellement des composés comprenant deux fonctions nitriles tels que le succinonitrile, l'adiponitrile.

Elle se rapporte plus particulièrement à la fabrication de composés comprenant des fonctions nitriles à partir de composés comprenant des fonctions carboxyliques, avantageusement, contenus dans des milieux ou jus fermentaires.

Les composés comprenant des fonctions nitriles et notamment les composés dinitriles tels que l'adiponitrile, le succinonitrile sont des produits importants pour la fabrication de composés amines ou de polymères, par exemple.

Ainsi, l'adiponitrile est un composé important utilisé comme composé intermédiaire dans la fabrication de l'hexaméthylène diamine, ou de l'epsilon-caprolactame. Ces deux composés sont des monomères utilisés dans la fabrication des polyamides, notamment du polyadipamide d'hexaméthylènediamine (PA 6,6) ou du polycaproamide (PA 6). L'hexaméthylène diamine est également utilisée pour la synthèse de diisocyanates, monomères importants dans la fabrication des polyuréthannes.

L'adiponitrile et le succinonitrile peuvent également être utilisés dans les procédés de fabrication des polyamides par réaction de condensation avec des monomère diacides.

De nombreux procédés de synthèse de l'adiponitrile ont été proposés. Ces procédés utilisent principalement comme matière première de départ des composés hydrocarbures issus du raffinage du pétrole. Ainsi, les procédés principaux de synthèse de l'adiponitrile sont l'hydrocyanation du butadiène, et l'ammoxydation du propane ou du propène.

Le document GB 797,945 décrit un procédé de production de dinitriles à partir de diacides. De l'ammoniac est utilisé pour la préparation du catalyseur. Les dinitriles produits ne sont pas l'adiponitrile. Les diacides ne sont pas d'origine bio.

In a également été exploité un procédé de fabrication d'adiponitrile par transformation d'acide adipique en adiponitrile en présence d'hydroxyde d'ammonium. Ce procédé est décrit notamment dans les brevets français n° 2028842, 2132849, 2144340.

Dans ce procédé, l'acide adipique utilisé comme matière première était issu d'un hydrocarbure tel que le cyclohexane obtenu au cours du raffinage du pétrole.

Compte tenu de l'épuisement de la ressource pétrolière, de nombreux travaux de recherche sont entrepris pour développer des procédés de synthèse de ces composés importants pour la fabrication de matériaux utilisés dans de nombreuses applications à partir de matières premières ou ressources dites renouvelables. Généralement ces ressources renouvelables sont constituées par de la matière végétale cultivée ou non telle que les arbres, les plantes comme la canne à sucre, le maïs, le manioc, le blé ou analogues.

Cette matière végétale est transformée par des procédés comprenant généralement plusieurs étapes mécaniques, chimiques et biologiques en composés appartenant à la famille des sucres tels que glucose, saccharose, fructose ou analogues. Les sucres ainsi obtenus sont ensuite transformés, avantageusement, par des procédés comme la fermentation en composés comprenant des fonctions organiques particulières. Ainsi, par un procédé de fermentation biologique, il est possible de transformer les sucres en composés comprenant des fonctions carboxyliques. Ces procédés de fermentation produisent des solutions aqueuses de composés organiques appelées jus fermentaires.

Un des objets de l'invention est de proposer un procédé de fabrication de composés comprenant au moins une fonction nitrile en utilisant comme matière première, préférentiellement une solution aqueuse d'un composé carboxylique et encore plus avantageusement les solutions aqueuses dites jus fermentaires obtenues par fermentation des sucres produits par la transformation des ressources renouvelables.

A cet effet, l'invention propose un procédé de fabrication de composés de formule générale I :

(NC)ₓ-(R₁)-(CN)_{y}

Dans laquelle
R₁ représente un groupement hydrocarboné linéaire ou ramifié, saturé ou insaturé, comprenant de 1 à 20 atomes de carbone et pouvant comprendre des hétéroatomes,
x et y représentent 0 ou 1 avec (x+y) égal à 1 ou 2.

Ce procédé consiste à faire réagir de l'ammoniac avec un sel d'un diacide organique de formule générale II

(H₄NOOC)ₓ-(R₁)-(COONH₄)_{y}

Dans laquelle x, y et R₁ ont la signification indiquée ci-dessus, en présence d'un catalyseur comprenant un orthophosphate de silicium cristallin.

Le procédé est mis en oeuvre à une température comprise entre 300°C et 450°C, préférentiellement entre 350°C et 425°C.

La solution aqueuse de composés de formule II est vaporisée avant sa mise en contact avec le catalyseur soit dans un dispositif de vaporisation soit par pulvérisation dans le flux d'ammoniac surchauffé. Ces deux modes de vaporisation de la solution aqueuse sont donnés uniquement à titre indicatif.

Selon une autre caractéristique de l'invention, le catalyseur comprend moins de 5% en poids d'orthophosphate de silicium amorphe.

Selon encore une autre caractéristique de l'invention, le composé de formule générale II est en solution aqueuse. Cette solution aqueuse est obtenue dans un procédé de transformation biologique d'une solution contenant des sucres issue elle-même d'un procédé de transformation ou d'extraction utilisant comme matières premières des matières essentiellement végétales constituant une ressource renouvelable.

Cette solution aqueuse issue d'un procédé de transformation biologique est obtenu par exemple par un procédé de fermentation de sucres. Ce milieu est généralement dénommé "jus fermentaire".

Ces jus fermentaires peuvent être utilisés directement ou après filtration pour séparer la matière biologique ou biomasse du milieu. A titre d'exemple de procédé de fabrication de jus fermentaires convenables comme matière première pour le procédé de l'invention, on peut citer les procédés décrits dans l'article de Varadarajan et al paru dans Biotechnol. Pro. 1999, 15, 845-854, ou l'article de Olson et al paru dans Applied Biochemistry and Biotechnology 2003, Vol 105-108, 843-851.

Les solutions aqueuses et/ou les jus fermentaires convenables pour le procédé de l'invention ont une concentration pondérale en composés de formule générale II généralement supérieure à 1 % en poids, de préférence comprise entre 1 % en poids et 30 % en poids, avantageusement entre 5 % en poids et 25 % en poids. Toutefois, la concentration maximale convenable est fixée par la limite de solubilité du composé de formule II dans l'eau à la température d'alimentation de la solution dans le dispositif de vaporisation utilisé pour alimenter la solution sur le catalyseur.

Cette concentration peut être obtenue directement par le procédé de fermentation biologique ou par concentration du jus fermentaire ou de la solution aqueuse, par exemple, par évaporation de l'eau. Avantageusement, le composé de formule générale II est choisi dans le groupe comprenant le succinate d'ammonium, l'adipate d'ammonium, le glutamate d'ammonium, le glutarate d'ammonium, les sels d'ammonium des diacides gras, l'hexadécanedioate d'ammonium ou analogues.

Un catalyseur convenable pour l'invention peut être avantageusement obtenu par imprégnation d'une silice par de l'acide phosphorique en solution aqueuse, puis calcination sous air, pour former l'orthophosphate de silicium. La température de calcination est avantageusement comprise entre 450°C et 800°C, par exemple comprise entre 450°C et 550°C. Un tel procédé de fabrication est notamment décrit dans la demande de brevet français n° 2810317.

Il est également possible d'utiliser des catalyseurs commercialisés par différentes sociétés tels que la société UOP. Toutefois, il peut être nécessaire de traiter ces catalyseurs commerciaux pour augmenter le taux de forme cristalline, par exemple, par un traitement thermique.

Le catalyseur est généralement sous forme solide par exemple en forme de billes, extrudés cylindriques, nid d'abeille ou analogue. Le catalyseur est disposé dans un réacteur sous forme d'un lit fixe à travers duquel est envoyé le jus fermentaire et l'ammoniac sous forme vapeur.

Le catalyseur de l'invention peut contenir également des éléments dopants ou des cocatalyseurs.

Selon une autre caractéristique de l'invention, le catalyseur utilisé pour la mise en oeuvre du procédé de l'invention peut être régénéré par traitement du lit de catalyseur avec de l'air à une température comprise entre 450 et 500°C pendant 10 à 20 heures. Le traitement de régénération peut être contrôlé par détection de la présence de CO₂ dans l'air en sortie de réacteur. Le traitement est arrêté quand l'absence de CO₂ dans l'air est constatée. Le catalyseur ainsi régénéré peut être utilisé pour une nouvelle mise en oeuvre du procédé de l'invention avec des performances catalytiques équivalentes.

Les vapeurs récupérées en sortie de réacteur son condensées pour récupérer le composé comprenant les fonctions nitriles. Ces composés peuvent être ensuite purifiés, par les techniques classiques telles que distillation, cristallisation, extraction ou analogue.

Le catalyseur est avantageusement activé notamment par traitement avec de l'ammoniac gazeux à une température comprise entre 350°C et 500°C, avant l'alimentation de la solution aqueuse de composé II.

D'autres détails, avantages de l'invention apparaitront plus clairement au vu des exemples donnés ci-dessous uniquement à titre indicatif.

La réaction est mise en oeuvre en phase gazeuse à une température voisine de 400°C et sous pression atmosphérique. Le catalyseur utilisé est constitué d'orthophosphate de silicium de formule Si₃(PO₄)₄.

Ce catalyseur est préparé par imprégnation d'une silice par de l'acide phosphorique à 85% puis calcination sous air à 500°C.

### Exemple 1 : Préparation de succinonitrile

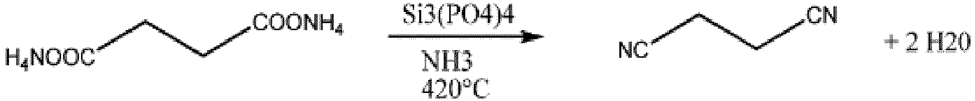

La matière première est une solution aqueuse contenant environ 10 à 20% en poids de succinate d'ammonium en solution dans l'eau.

Cette solution aqueuse contient d'autres acides et diacides comprenant moins d'atomes de carbone et appelés « acides inférieurs ».

La composition de la solution aqueuse utilisée correspond à la composition d'une solution ou jus fermentaire issu d'un procédé de fermentation d'un milieu contenant des sucres issus du traitement d'une matière végétale.

La solubilité des sels d'ammonium de ces acides augmente beaucoup avec la température, aussi il peut être intéressant d'utiliser une solution chaude pour éviter des problèmes de cristallisation.

Dans un réacteur en verre de 22 mm de diamètre , on introduit successivement 3ml de poudre de verre 5 ml (4,0 g) de catalyseur Si₃(P0₄)₄ sous forme d'extrudés 3 ml de poudre de verre

Le lit catalytique est activé d'une part par un traitement à 500°C sous un courant d'air de 3 l/h pendant environ 15 heures, puis après abaissement de la température à 420°C, remplacement de l'air par un flux de NH₃ selon un débit de 1,5 l/h.

A l'aide d'un pousse seringue, une solution aqueuse de succinate d'ammonium à une concentration de 15% en poids est injectée, selon un débit de 6ml/h. Dans ces conditions, le rapport molaire NH₃/Succinate d'ammonium est égal à 10.

Les condensats sont récupérés et analysés par Chromatographie en Phase Gazeuse (CPG).

Après 4 heures d'injection de solution aqueuse, le rendement de transformation en succinonitrile par rapport au succinate d'ammonium introduit est de 65%

### Exemple2 : Préparation de succinonitrile ex acide succinique

L'exemple 1 est répété mais en utilisant une solution aqueuse d'acide succinique à 20% en poids.

Le lit catalytique est identique à l'essai précédent. Après activation, on fait passer sur le lit catalytique un courant d'ammoniac de 2 l/h et on injecte conjointement la solution aqueuse d'acide succinique selon un débit de 4 ml/h . Dans ces conditions, le rapport molaire NH₃/acide succinique est de 10. Les condensats sont récupérés et analysés par CPG

Apres 5 heures d'injection, le rendement en succinonitrile est de 70%

### Exemple 3 : Préparation d'adiponitrile.

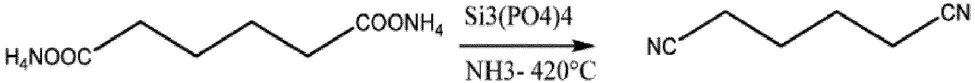

L'exemple 1 est répété en remplaçant la solution de succinate d'ammonium par une solution d'adipate d'ammonium à 15% en poids. Un flux de NH₃ de 1,5 /h est injecté sur le lit catalytique et la solution aqueuse chaude d'adipate d'ammonium est injectée selon un débit de 4ml/h.

Après 4 heures d'injection, le rendement de transformation de l'adipate d'ammonium en adiponitrile est de 72%.

### Exemple 4 : Préparation de succinonitrile avec catalyseur régénéré.

Quand les performances du lit catalytique deviennent inférieures à l'optimum économique, celui-ci peut être régénéré selon la procédure suivante décrite uniquement à titre d'exemple.

L'alimentation sur le lit catalytique en ammoniac et solution aqueuse de carboxylate d'ammonium est arrêtée. Le lit catalytique maintenu sous courant de d'azote est refroidi à température ambiante. Le courant d'azote est remplacé par un courant de 3l/h d'air et le lit catalytique est chauffé progressivement jusqu'à 500°C. Le traitement sous air à 500°C est maintenu pendant environ 15h. La présence de CO₂ dans l'air sortant est détectée et le traitement est poursuivi jusqu'à ce que le CO₂ ne soit plus détecté dans le flux de sortie.

La température du lit catalytique est ramené à 420°C en remplaçant le débit d'air par un courant d'azote de 3l/h. A 420°C, le courant d'azote est remplacé progressivement par l'ammoniac. Le catalyseur est alors régénéré et un essai de production de succinonitrile a été réalisé selon le mode opératoire décrit à l'exemple 1. Le rendement de transformation obtenu est identique.

## Revendications

1. Procédé de fabrication de composés de formule générale I
(NC)ₓ-R₁(CN)_{y}
dans laquelle :
R₁ représente un groupement hydrocarboné linéaire ou ramifié, saturé ou insaturé, comprenant de 1 à 20 atomes de carbone et pouvant comprendre des hétéroatomes,
x, y est égal à 0 ou 1 avec (x+y) égal à 1 ou 2
consistant à faire réagir, en phase vapeur, l'ammoniac avec un composé de formule générale II
(H₄NOOC)ₓ-(R₁)-(COONH₄)_{y}
en présence d'un catalyseur comprenant un orthophosphate de silicium de formule Si₃(PO₄)₄, à une température comprise entre 300°C et 450°C,
**caractérisé en ce que** le composé de formule générale II est en solution aqueuse, ladite solution aqueuse étant un jus fermentaire issu d'un procédé de transformation biologique d'un milieu contenant des sucres, et étant utilisé directement ou après filtration de la matière biologique ou biomasse du milieu.

2. Procédé selon la revendication 1, **caractérisé en ce que** la concentration du composé de formule générale II dans la solution aqueuse est supérieure à 1% en poids.

3. Procédé selon la revendication 2, **caractérisé en ce que** la concentration du composé de formule générale II dans la solution aqueuse est comprise entre 1% et 30% en poids, de préférence entre 1% et 25% en poids.

4. Procédé selon l'une quelconque des revendications précédentes, **caractérisé en ce que** le milieu issu de la transformation biologique utilisé est obtenu après séparation de la biomasse.

5. Procédé selon l'une des revendications 1 à 4, **caractérisé en ce que** le composé de formule générale II est choisi dans le groupe comprenant le succinate d'ammonium, l'adipate d'ammonium, le glutamate d'ammonium, le glutarate d'ammonium, les sels d'ammonium des diacides gras, l'hexadécanedioate d'ammonium ou analogues.

6. Procédé selon l'une des revendications 1 à 5, **caractérisé en ce que** la réaction est réalisée dans un réacteur comprenant un lit catalytique fixe.

7. Procédé selon l'une des revendications 1 à 6, **caractérisé en ce que** le catalyseur est obtenu par imprégnation d'une silice avec de l'acide phosphorique, et calcination sous air.

8. Procédé selon la revendication 7 **caractérisé en ce que** la calcination est réalisée à une température comprise entre 400°C et 800°C.

9. Procédé selon l'une des revendications précédentes, **caractérisé en ce que** le composé de formule générale I est choisi dans le groupe comprenant l'adiponitrile, le succinonitrile.

## Patentansprüche

1. Verfahren zur Herstellung von Verbindungen der allgemeinen Formel I
(NC)ₓ-R₁(CN)_{y}
in der:
R₁ für eine gesättigte oder ungesättigte, lineare oder verzweigte Kohlenwasserstoffgruppe, die 1 bis 20 Kohlenstoffatome umfasst und Heteroatome umfassen kann, steht,
x und y gleich 0 oder 1 sind, wobei (x+y) gleich 1 oder 2 ist,
bei dem man Ammoniak in der Gasphase in Gegenwart eines Katalysators, der ein Siliciumorthophosphat der Formel Si₃(PO₄)₄ umfasst, bei einer Temperatur zwischen 300°C und 450°C mit einer Verbindung der allgemeinen Formel II
(H₄NOOC)ₓ-(R₁)-(COONH₄)_{y}
umsetzt,
**dadurch gekennzeichnet, dass** die Verbindung der allgemeinen Formel II in wässriger Lösung vorliegt und die wässrige Lösung eine aus einem Verfahren der biologischen Transformation eines Zucker enthaltenden Mediums stammende Fermentationsbrühe ist und direkt oder nach Abfiltrieren des biologischen Materials bzw. der Biomasse von dem Medium verwendet wird.

2. Verfahren nach Anspruch 1, **dadurch gekennzeichnet, dass** die Konzentration der Verbindung der allgemeinen Formel II in der wässrigen Lösung größer als 1 Gew.-% ist.

3. Verfahren nach Anspruch 2, **dadurch gekennzeichnet, dass** die Konzentration der Verbindung der allgemeinen Formel II in der wässrigen Lösung zwischen 1 und 30 Gew.-%, vorzugsweise zwischen 1 und 25 Gew.-%, liegt.

4. Verfahren nach einem der vorhergehenden Ansprüche, **dadurch gekennzeichnet, dass** das aus der biologischen Transformation stammende verwendete Medium nach Abtrennung der Biomasse erhalten wird.

5. Verfahren nach einem der Ansprüche 1 bis 4, **dadurch gekennzeichnet, dass** die Verbindung der allgemeinen Formel II aus der Gruppe bestehend aus Ammoniumsuccinat, Ammoniumadipat, Ammoniumglutamat, Ammoniumglutarat, Ammoniumsalzen von Difettsäuren, Ammoniumhexadecandioat oder dergleichen ausgewählt wird.

6. Verfahren nach einem der Ansprüche 1 bis 5, **dadurch gekennzeichnet, dass** die Umsetzung in einem Reaktor mit einem Katalysatorfestbett durchgeführt wird.

7. Verfahren nach einem der Ansprüche 1 bis 6, **dadurch gekennzeichnet, dass** der Katalysator durch Imprägnieren eines Siliciumdioxids mit Phosphorsäure und Calcinieren an der Luft erhalten wird.

8. Verfahren nach Anspruch 7, **dadurch gekennzeichnet, dass** die Calcinierung bei einer Temperatur zwischen 400°C und 800°C durchgeführt wird.

9. Verfahren nach einem der vorhergehenden Ansprüche, **dadurch gekennzeichnet, dass** die Verbindung der allgemeinen Formel I aus der Gruppe bestehend aus Adiponitril und Succinonitril ausgewählt wird.

## Claims

1. Process for preparing compounds of general formula I
(NC)ₓ-R₁(CN)_{y}
in which:
R₁ represents a saturated or unsaturated, linear or branched hydrocarbon group containing 1 to 20 carbon atoms and possibly containing heteroatoms,
x and y are 0 or 1, with (x+1) being 1 or 2,
which comprises reacting ammonia with a compound of general formula II
(H₄NOOC)ₓ-(R₁)-(COONH₄)_{y}
in vapour phase in the presence of a catalyst comprising a silicon orthophosphate of formula Si₃(PO₄)₄, at a temperature of between 300°C and 450°C,
**characterized in that** the compound of general formula II is in aqueous solution, said aqueous solution being a fermentation liquor obtained from a process of biological transformation of a medium containing sugars, and being used directly or after filtration to separate the biological material or biomass from the medium.

2. Process according to Claim 1, **characterized in that** the concentration of the compound of general formula II in the aqueous solution is greater than 1% by weight.

3. Process according to Claim 2, **characterized in that** the concentration of the compound of general formula II in the aqueous solution is between 1% and 30% by weight, preferably between 1% and 25% by weight.

4. Process according to any of the preceding claims, **characterized in that** the medium obtained from the biological transformation that is used is obtained after separation from the biomass.

5. Process according to any of Claims 1 to 4, **characterized in that** the compound of general formula II is selected from the group comprising ammonium succinate, ammonium adipate, ammonium glutamate, ammonium glutarate, ammonium salts of fatty diacids, ammonium hexadecanedioate or the like.

6. Process according to any of Claims 1 to 5, **characterized in that** the reaction is performed in a reactor comprising a fixed catalyst bed.

7. Process according to any of Claims 1 to 6, **characterized in that** the catalyst is obtained by impregnating a silica with phosphoric acid and calcining in air.

8. Process according to Claim 7, **characterized in that** the calcining is performed at a temperature of between 400°C and 800°C.

9. Process according to any of the preceding claims, **characterized in that** the compound of general formula I is selected from the group comprising adiponitrile and succinonitrile.
